# EUROPEAN PATENT SPECIFICATION

(11) **EP 4 223 419 B1**
(45) Date of publication and mention of the grant of the patent: **02.10.2024**
(21) Application number: 22213203.7
(22) Date of filing: 13.12.2022
(51) Int. Cl.: B01L 9/06, A61B 10/00

(54) **POUCH FOR IN-VITRO DIAGNOSTIC ITEMS**
BEUTEL FÜR IN-VITRO-DIAGNOSEARTIKEL
POCHE POUR ARTICLES DE DIAGNOSTIC IN VITRO

(30) Priority: 04.02.2022 KR 20220014587
(43) Date of publication of application: 09.08.2023
(73) Proprietor: Park, Jae Ku, Gwangmyeong-si 14221 (KR)
(72) Inventor: PARK, Jae Ku, 14221 Gwangmyeong-si (KR); KIM, Seung Hoon, Hanam-si (KR)
(74) Representative: Jacobacci Coralis Harle

(56) References cited:
- WO-A1-2008/016268
- WO-A1-2018/151595
- KR-U- 20180 002 522
- US-A1- 2014 243 706
- US-B1- 6 291 171
- US-B2- 10 034 660

## Description

### FIELD OF THE INVENTION

The present invention relates to a pouch for in-vitro diagnostic items, and more particularly, to a pouch used to store items used for in-vitro diagnosis.

### DESCRIPTION OF THE PRIOR ART

In-vivo diagnosis has been widely used in the past to examine the inside of a human body and identify disease states using endoscopy, computed tomography (CT), magnetic resonance tomography (MRI), and the like. In recent years, in-vitro diagnostic technology for separating test materials such as blood, saliva, and feces (hereinafter referred to as specimen) from the human body and then examining human diseases based on these samples is mainly used.

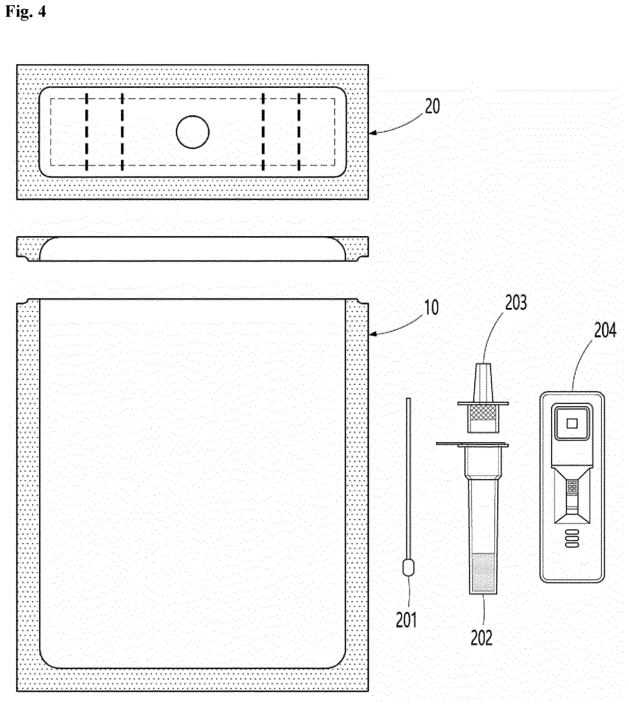

In-vitro diagnosis includes the steps of collecting a sample from the human body by using a collection tool such as a pipette or a cotton swab 201(refer to FIG. 4); extracting the sample by immersing the collection tool in a sample diluent stored in a buffer 202(refer to FIG. 4); and passing the sample diluent containing the extracted sample through a filter cap 203 (refer to FIG. 4) to drop it on a dripping site of a device 204 (refer to FIG. 4).

The device 204 is usually configured to determine whether there is a disease by applying a qualitative or quantitative technique to the sample falling on the dripping site, and then display the disease state.

The filter cap 203 has a filter installed therein, and is detachably coupled to the upper end of the buffer 202.

Hereinafter, for convenience of description, at least one of the collection tool 201, the buffer 202, the filter cap 203, and the device 204 used for in-vitro diagnosis is referred to as diagnosis item.

In the stage of performing the in-vitro diagnosis, a means for supporting the buffer 202 in an upright state is required so that the sample diluent is not spilled.

Hereinafter, for convenience of description, the means for supporting the buffer 202 in an upright state is referred to as buffer support means. Such buffer support means is not included in the diagnosis item.

A pouch has been devised and used to store the diagnosis item. Hereinafter, the pouch for storing the diagnosis item is referred to as pouch for in-vitro diagnostic items.

The diagnosis item stored in the pouch for in-vitro diagnostic items may be taken out and used before performing in-vitro diagnosis, and the used diagnosis item may be put back into the pouch for in-vitro diagnostic items and discarded.

Such a pouch stores the diagnosis item in the quantity required for one or multiple times in-vitro diagnoses.

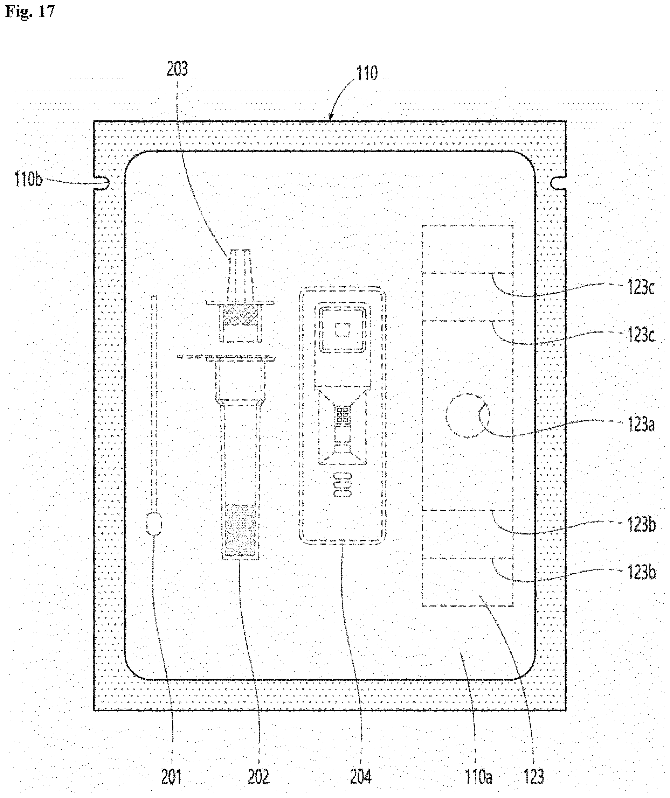

FIG. 17 is a front view of a conventional pouch for in-vitro diagnostic items.

The conventional pouch for in-vitro diagnostic items has a pocket portion of the diagnosis item 110 with a storage space of the diagnosis item 110a formed therein, as shown in FIG. 17.

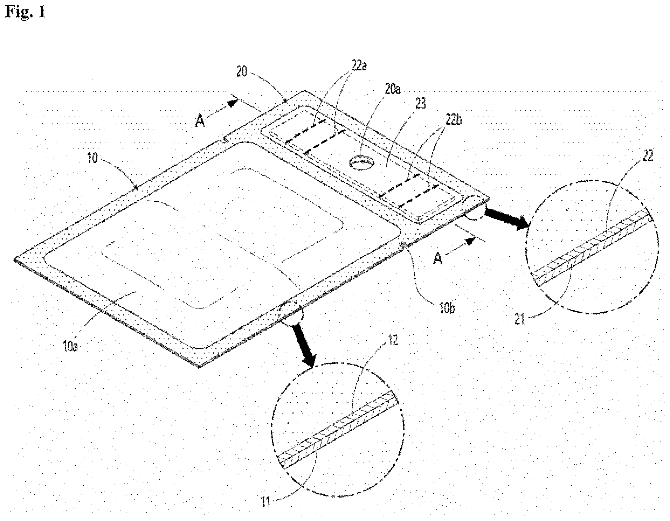

The pocket portion of the diagnosis item 110 has a rectangular back sheet of the diagnosis item pocket (refer to reference number 11 in FIG. 1) and a rectangular front sheet of the diagnosis item pocket coupled to the back sheet of the diagnosis item pocket (refer to reference number 12 in FIG. 1).

A pair of cut-out grooves 110b are formed to face each other in the back sheet of the diagnosis item pocket and the front sheet of the diagnosis item pocket.

The front sheet of the diagnosis item pocket is superimposed on the back sheet of the diagnosis item pocket.

A bending member for forming supporter 123 is formed in a shape of a plate body where a buffer support hole 123a is formed penetratingly in the center along a thickness direction.

On the outer surface of the bending member for forming supporter 123 are marked a left bending position line 123b indicating the left bending motion position and a right bending position line 123c indicating the right bending motion position.

A method of manufacturing the conventional pouch for in-vitro diagnostic items having the above-described configuration is described as follows.

First, a back sheet of the diagnosis item pocket and a front sheet of the diagnosis item pocket are prepared. PET, PE, aluminum, etc. may be used for the back sheet of the diagnosis item pocket and the front sheet of the diagnosis item pocket.

Next, the diagnosis item and the bending member for forming supporter 123 are placed on an area where the storage space of the diagnosis item 110a is will be formed among the upper surface of the back sheet of the diagnosis item pocket.

Next, the front sheet of the diagnosis item pocket is placed on top of the back sheet of the diagnosis item pocket.

Next, the edge area of the front sheet of the diagnosis item pocket and the back sheet of the diagnosis item pocket are coupled by using thermal bonding technique.

Here, since the thermal bonding technique is widely known, a detailed description thereof is omitted.

A method of performing in-vitro diagnosis by using the conventional pouch for in-vitro diagnostic items is described as follows.

First, by using scissors the pocket portion of the diagnosis item 110 is cut off along the direction in which the cut-out grooves 110b are connected.

Next, the diagnosis item and the bending member for forming supporter 123 stored in the storage space of the diagnosis item 110a are taken out.

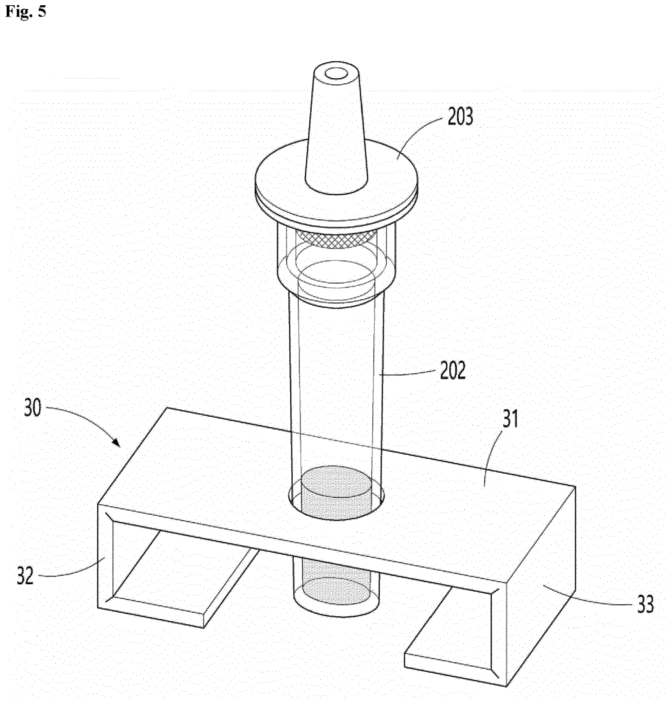

Next, the bending member for forming supporter 123 is bent along the left bending position line 123b and the right bending position line 123c to form a buffer supporter (refer to reference number 30 in FIG. 5).

Next, in-vitro diagnosis is performed by using the diagnosis item.

During in-vitro diagnosis, the buffer 202 may be provided in an upright state on the buffer supporter.

The bending member for forming supporter 123 may be stored in a location other than the storage space of the diagnosis item 110a.

However, according to the conventional pouch for in-vitro diagnostic items, since the bending member for forming supporter 123 is stored in the storage space of the diagnosis item 110a together with the diagnosis item, the buffer supporter must be manufactured separately from the pouch. In addition, the buffer supporter must be accommodated in the pocket portion of the diagnosis item 110 when manufacturing the pouch, and the size of the storage space of the diagnosis item 110a is increased to accommodate the buffer supporter.

### PRIOR ART

Patent Document: a Korean Patent Publication No. 10-0735080 (Publication date: July 3, 2007).

US 6 291 171 B1 discloses a pouch as defined in the preamble of claim 1.

### SUMMARY OF THE INVENTION

Therefore, it is an object of the present invention to provide a pouch for in-vitro diagnostic items that does not need to manufacture a buffer supporter separately from the pouch, to accommodate the buffer supporter in the pocket portion of the diagnosis item when manufacturing the pouch, and to increase the size of the storage space of the diagnosis item in order to accommodate the buffer supporter.

The invention is defined in claim 1. Additional embodiments are defined in the dependent claims.

### EFFECTS OF THE INVENTION

Therefore, according to the present invention, there is no need to manufacture the buffer supporter separately from the pouch, to accommodate the buffer supporter in the pocket portion of the diagnosis item when manufacturing the pouch, and to increase the size of the storage space of the diagnosis item in order to accommodate the buffer supporter.

### BRIEF DESCRIPTION OF THE DRAWINGS

FIG. 1 is a perspective view of a pouch for in-vitro diagnostic items according to a first embodiment of the present invention;
FIG. 2 is a cross-sectional view taken along line A-A of FIG. 1;
FIG. 3 is a perspective view of a bending member for forming supporter according to a first embodiment of the present invention;
FIG. 4, 5 and 6 are views each showing a method for performing in-vitro diagnosis by using a pouch for in-vitro diagnostic items according to the first embodiment of the present invention;
FIG. 7 is a front view of a pouch for in-vitro diagnostic items according to a second embodiment of the present invention;
FIG. 8 is a perspective view of a bending member for forming supporter according to a second embodiment of the present invention;
FIG. 9 is a cross-sectional view taken along line B-B of FIG. 7;
FIG. 10 is a rear view of a pouch for in-vitro diagnostic items according to a third embodiment of the present invention;
FIG. 11 is a perspective view of a bending member for forming supporter according to a fourth embodiment of the present invention;
FIG. 12 is a perspective view of a pouch for in-vitro diagnostic items according to a fifth embodiment of the present invention;
FIG. 13 is a cross-sectional view taken along line C-C of FIG. 12;
FIG. 14 is a front view of a pouch for in-vitro diagnostic items according to a sixth embodiment of the present invention;
FIG. 15 is a front view of a pouch for in-vitro diagnostic items according to a seventh embodiment of the present invention;
FIG. 16 is a perspective view of a pouch for in-vitro diagnostic items according to an eighth embodiment of the present invention;
FIG. 17 is a front view of a conventional pouch for in-vitro diagnostic items.

### DETAILED DESCRIPTION OF THE EXEMPLARY EMBODIMENTS

Hereinafter, the present invention will be described in detail with reference to the accompanying drawings.

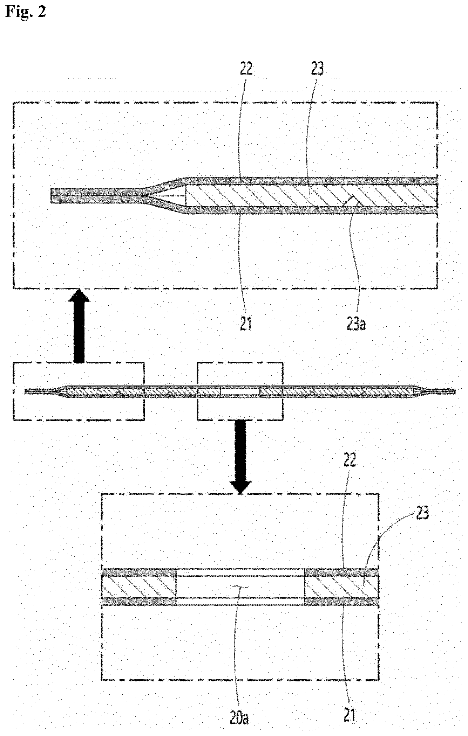

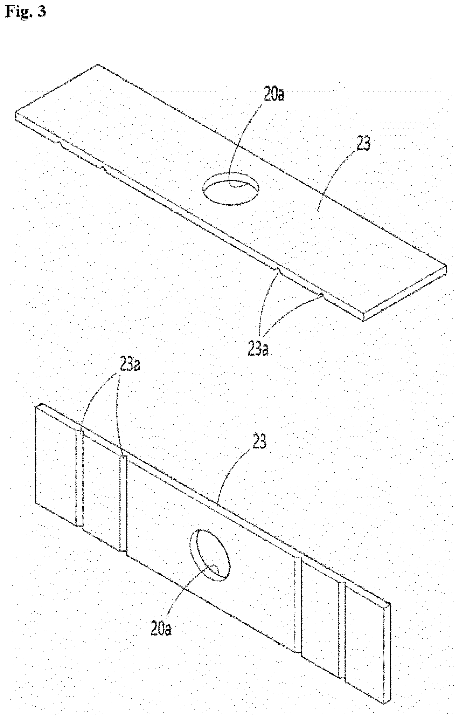

Fig. 1 is a perspective view of a pouch for in-vitro diagnostic items according to a first embodiment of the present invention, Fig. 2 is a cross-sectional view taken along line A-A of Fig. 1, and Fig. 3 is a perspective view of a bending member for forming supporter according to a first embodiment of the present invention.

The pouch for in-vitro diagnostic items according to the first embodiment of the present invention includes, as shown in these drawings, a pocket portion of the diagnosis item 10 having a storage space of the diagnosis item 10a formed therein, and a buffer supporter formation portion 20 formed by extending in a plate shape from one side of the pocket portion of the diagnostic item 10.

The pocket portion of the diagnosis item 10 has a rectangular back sheet of the diagnosis item pocket 11 and a rectangular front sheet of the diagnosis item pocket 12 coupled to the back sheet of the diagnosis item pocket 11.

The front sheet of the diagnosis item pocket 12 is coupled to be superimposed on the back sheet of the diagnosis item pocket 11.

The storage space of the diagnosis item 10a is formed between the back sheet of the diagnosis item pocket 11 and the front sheet of the diagnosis item pocket 12.

A pair of cut-out grooves 10b are formed to face each other in the back sheet of the diagnosis item pocket 11 and the front sheet of the diagnosis item pocket 12.

The buffer supporter formation portion 20 has a rectangular back sheet of the buffer supporter 21 formed by extending from one side of the back sheet of the diagnosis item pocket 11, and a rectangular front sheet of the buffer supporter 22 formed by extending from the front sheet of the diagnosis item pocket 12. and a bending member for forming supporter 23 provided between the inner surface of the back sheet of the buffer supporter 21 and the inner surface of front sheet of the buffer supporter 22.

The front sheet of the buffer supporter 22 is coupled to be superimposed on the back sheet of the buffer supporter 21.

On the outer surface of the front sheet of the buffer supporter 22 are marked a left bending position line 22a indicating a left bending motion position and a right bending position line 22b indicating a right bending motion position.

The bending member for forming supporter 23 is formed in a shape of a rectangular plate body.

In the bending member for forming supporter 23, concave-shaped bending auxiliary grooves 23a are formed each at the point where a left bending motion occurs and at the point where a right bending motion occurs.

The bending auxiliary grooves 23a serve to assist in the bending of the bending member for forming supporter 23.

A buffer support hole 20a is formed so as to penetrate the back sheet of the buffer supporter 21, the front sheet of the buffer supporter 22 and the bending member for forming supporter 23 in a thickness direction.

A method of manufacturing the pouch for in-vitro diagnostic items according to the first embodiment of the present invention having the above-described configuration is described as follows. For convenience of explanation, it is assumed that the bending member for forming supporter 23 has been manufactured. The bending member for forming supporter 23 may be manufactured by using synthetic resin, paper, wire, metal sheet, or the like.

First, it is necessary to prepare a pouch back sheet in which the back sheet of the buffer supporter 21 is connected to the back sheet of the diagnosis item pocket 11 and a pouch front sheet in which the front sheet of the buffer supporter 22 is connected to the front sheet of the diagnosis item pocket 12. A left bending position line 22a and a right bending position line 22b are marked at the front sheet of the buffer supporter 22. The back sheet of the buffer supporter 21, the front sheet of the buffer supporter 22, the back sheet of the diagnosis item pocket 11, and the front sheet of the diagnosis item pocket 12 may be manufactured by using PET, PE, aluminum, or the like. For example, the back sheet of the buffer supporter 21, the front sheet of the buffer supporter 22, the back sheet of the diagnosis item pocket 11 and the front sheet of the diagnosis item pocket 12 may be manufactured in a triple layer structure comprised of an aluminum film, a PET film coupled to the outer surface of the aluminum film and a PE coating layer formed on the inner surface of the aluminum film.

Next, the diagnosis item is placed on an area where the storage space of the diagnosis item 10a will be formed among the upper surface of the back sheet of the diagnosis item pocket 11.

Next, the bending member for forming supporter 23 is placed on the upper surface of back sheet of the buffer supporter 21.

Next, the edge area of the pouch back sheet and the pouch front sheet and the area between the bending member for forming supporter 23 and the diagnosis item are coupled together by using thermal bonding technique.

A method of performing in-vitro diagnosis by using the pouch for in-vitro diagnostic items according to the first embodiment of the present invention is described with reference to FIGS. 4, 5 and 6 as follows.

First, by using scissors or the like, the pocket portion of the diagnosis item 10 is cut off along a direction in which the cut-out groove 10b is connected. After cutting off the pocket portion of the diagnosis item 10, it is also desirable to cut off a pocket portion remaining in the buffer supporter formation portion 20.

Next, the diagnosis item stored in the storage space of the diagnosis item 10a is taken out.

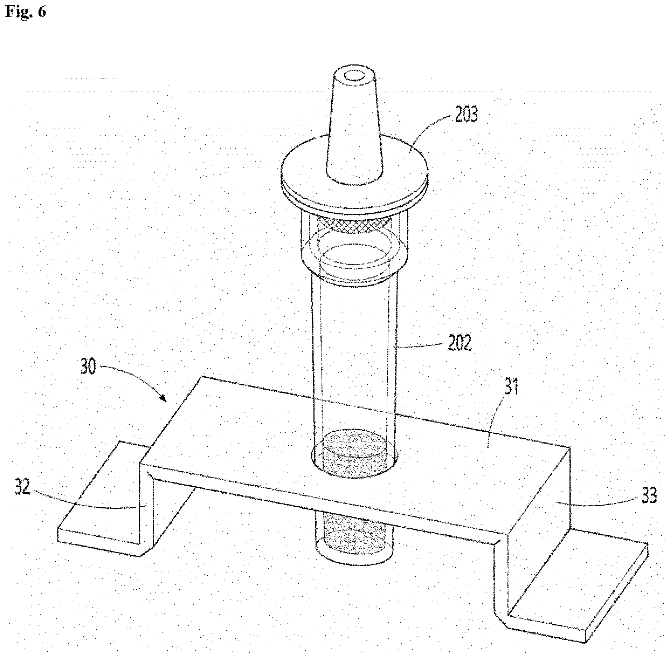

Next, the buffer supporter 30 is formed by bending the front sheet of the buffer supporter 22, the back sheet of the buffer supporter 21, and the bending member for forming supporter 23 along the left bending position line 22a and the right bending position line 22b. The buffer supporter 30 may be formed in a shape shown in FIG. 5 or FIG. 6.

The buffer supporter 30 includes a buffer-mounted shelf 31 for mounting the buffer 202, a left leg portion for supporting shelf 32 to support the left end of the buffer-mounted shelf 31 against the bottom surface, and a right leg portion for supporting shelf 33 to support the right end of the buffer-mounted shelf 31 against the bottom surface.

Next, in-vitro diagnosis is performed by using the diagnosis item.

During in-vitro diagnosis, the buffer 202 may be provided in an upright state on the buffer supporter 30.

When a plurality of buffers 202 are stored in the pocket portion of the diagnosis item 10, each buffer 202 may be sequentially provided in an upright state on the buffer supporter 30.

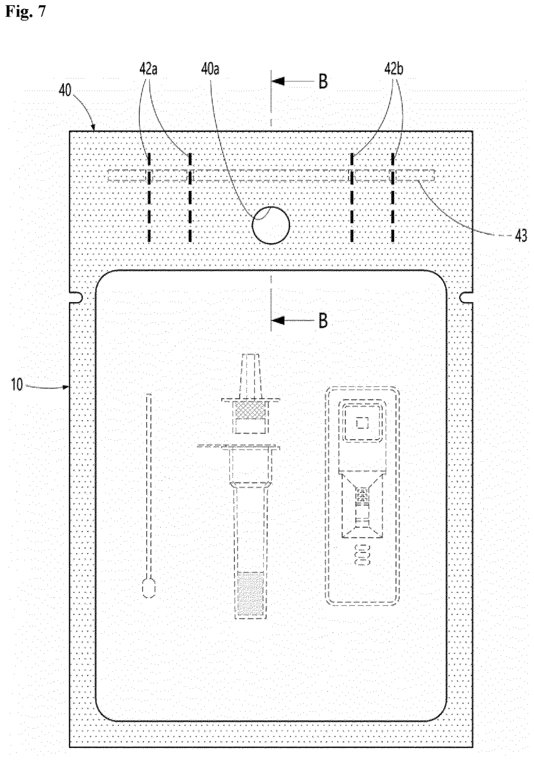

On the other hand, in the above embodiment, the bending member for forming supporter 23 is formed in a plate shape, but the present invention may be implemented by forming a bending member for forming supporter 43 in a straight shape as shown in FIG. 7.

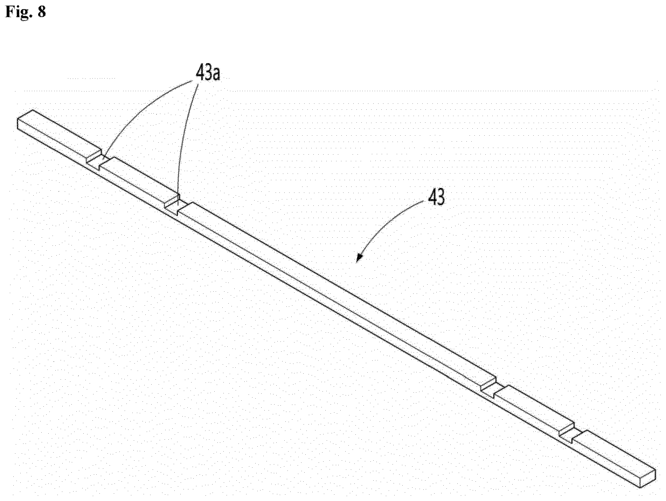

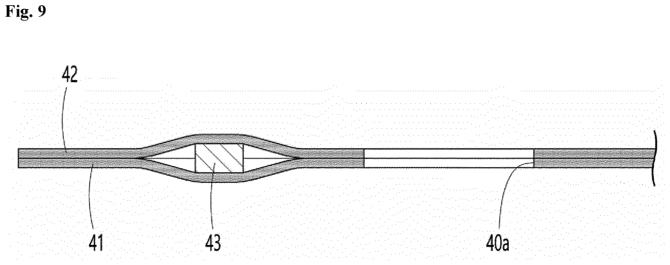

FIG. 7 is a front view of a pouch for in-vitro diagnostic items according to a second embodiment of the present invention, FIG. 8 is a perspective view of a bending member for forming supporter according to a second embodiment of the present invention, and FIG. 9 is a cross-sectional view taken along line B-B of FIG. 7.

In the case of the pouch for in-vitro diagnostic items according to the second embodiment of the present invention, the bending member for forming supporter 43 is provided between the inner surface of the back sheet of the buffer supporter 41 and the inner surface of the front sheet of the buffer supporter 42, the buffer support hole 40a is formed in the back sheet of the buffer supporter 41 and the front sheet of the buffer supporter 42.

In the bending member for forming supporter 43, bending auxiliary grooves 43a are formed.

And in the above embodiments, the bending member for forming supporter is provided between the inner surface of the back sheet of the buffer supporter and the inner surface of the front sheet of the buffer supporter, but the bending member for forming supporter may be provided on the outer surface of the back sheet of the buffer supporter or the outer surface of front sheet of the buffer supporter.

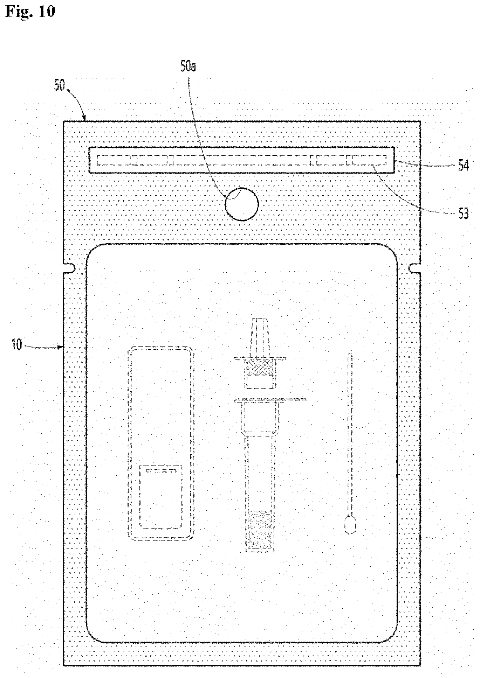

FIG. 10 is a rear view of a pouch for in-vitro diagnostic items according to a third embodiment of the present invention.

In the case of the pouch for the in-vitro diagnostic items according to the third embodiment of the present invention, a bending member for forming supporter 53 is provided on the outer surface of a back sheet of the buffer supporter. The bending member for forming supporter 53 may be fixed on the outer surface of the back sheet of the buffer supporter by using a fixing pad 54.

Meanwhile, in the above embodiments, the bending member for forming supporter is formed in a shape of a plate body or straight line, but the bending member for forming supporter may be formed in another shape.

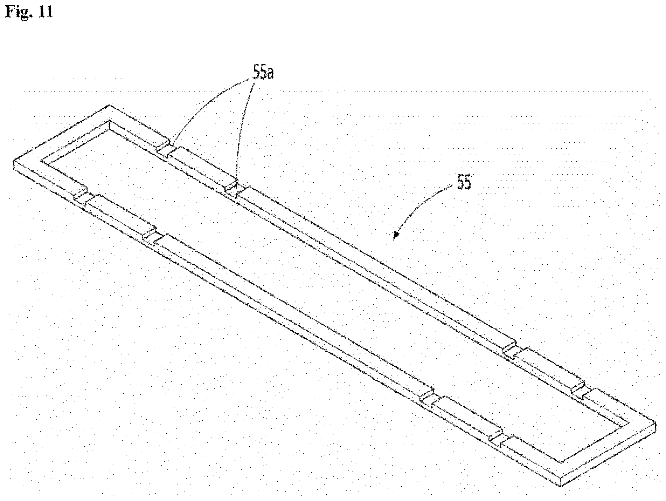

FIG. 11 is a perspective view of a bending member for forming supporter according to a fourth embodiment of the present invention.

A bending member for forming supporter 55 according to the fourth embodiment of the present invention is formed in a rectangular shape and bending auxiliary grooves 55a are formed in it.

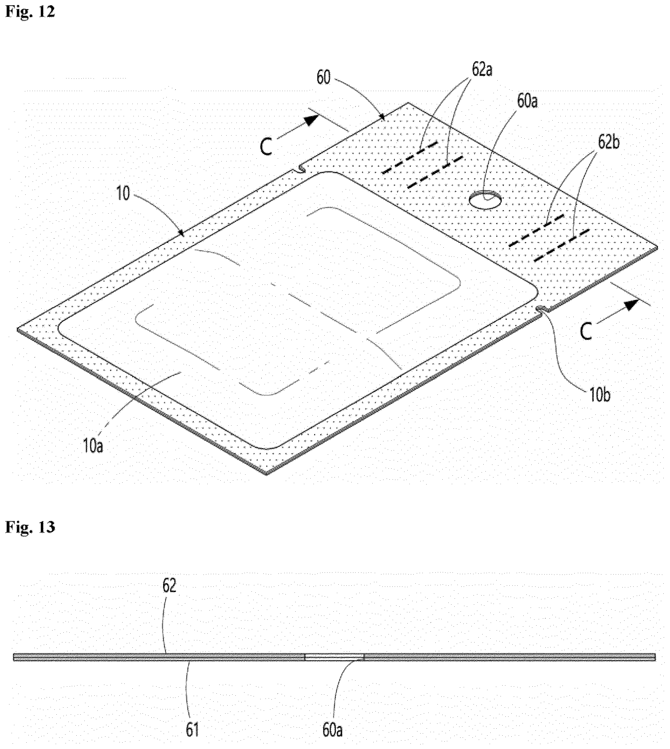

In addition, in the above-described embodiments, the buffer supporter formation portion is configured to include the bending member for forming supporter, but, as shown in FIG. 12, the present invention may be implemented by forming the buffer supporter formation portion in a structure not having the bending member for forming supporter.

FIG. 12 is a perspective view of a pouch for in-vitro diagnostic items according to a fifth embodiment of the present invention, and FIG. 13 is a cross-sectional view taken along line C-C of FIG. 12.

A buffer support formation portion 60 of the pouch for in-vitro diagnostic items according to the fifth embodiment of the present invention has a rectangular back sheet of the buffer supporter 61 formed by extending from one side of the back sheet of the diagnosis item pocket (refer to reference number 11 in FIG. 1), and a rectangular front sheet of the buffer supporter 62 formed by extending from one side of the front sheet of the diagnosis item pocket (refer to reference number 12 in FIG. 1).

A buffer support hole 60a is formed so as to penetrate the back sheet of the buffer supporter 61 and the front sheet of the buffer supporter 62 in a thickness direction.

Also, in the above-described embodiments, the buffer supporter formation portion is formed by extending from the horizontal side of the pocket portion of the diagnosis item 10, but the present invention may be implemented by extending the buffer supporter formation portion from another side of the pocket portion of the diagnosis item 10.

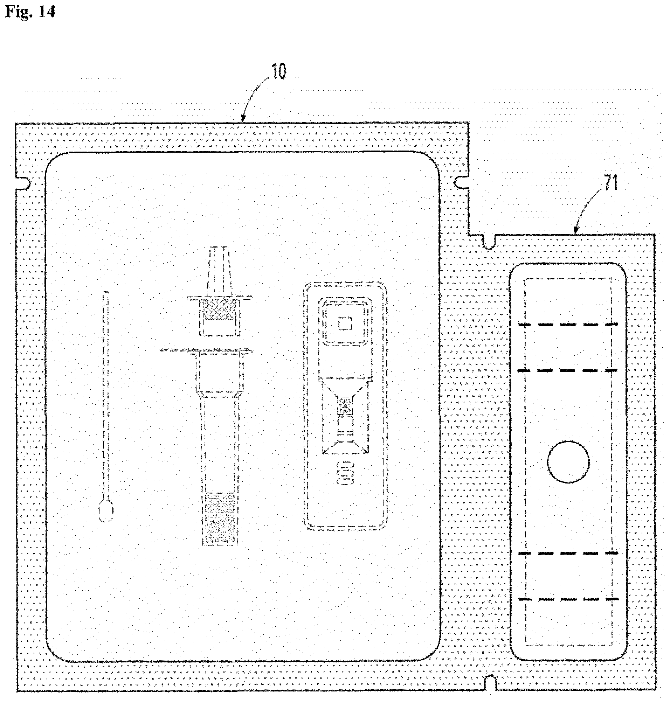

FIG. 14 is a front view of a pouch for in-vitro diagnostic items according to a sixth embodiment of the present invention.

In the case of the pouch for in-vitro diagnostic items according to the sixth embodiment of the present invention, a buffer supporter formation portion 71 is formed by extending from a longitudinal side of the pocket portion of the diagnosis item 10.

In addition, the present invention may be implemented by forming the buffer supporter formation portion so that the bending direction of the buffer supporter formation portion in a state connected to the pocket portion of the diagnosis item 10 is different from that described in the above embodiments.

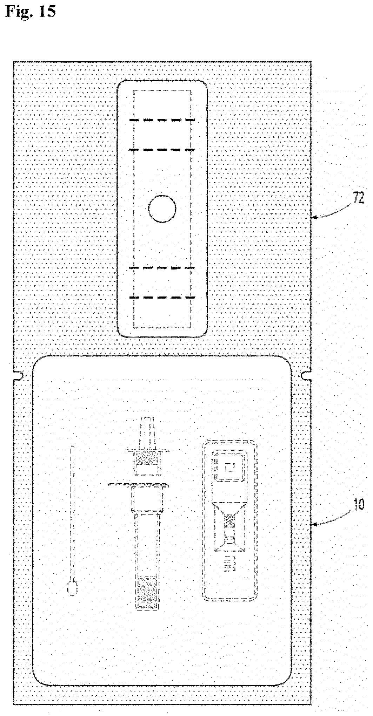

FIG. 15 is a front view of a pouch for in-vitro diagnostic items according to a seventh embodiment of the present invention.

The bending direction of a buffer supporter formation portion 72 according to the seventh embodiment of the present invention is perpendicular to that of the buffer supporter formation portion 20 according to the first embodiment of the present invention.

In addition, although the above-described embodiments are configured to have one buffer supporter formation portion, it is possible to implement the present invention by configuring it to have a plurality of buffer supporter formation portions.

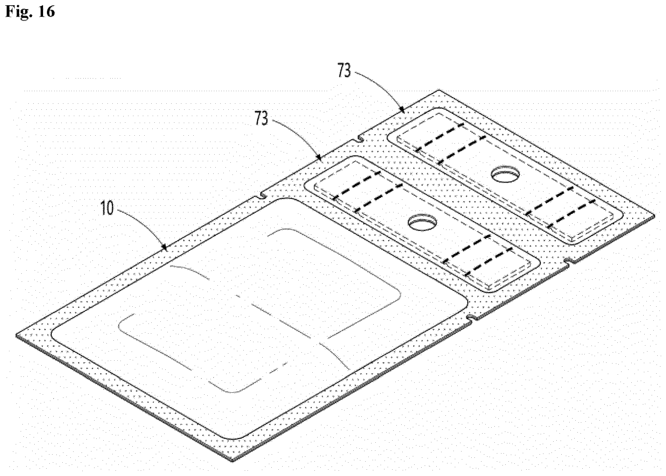

FIG. 16 is a perspective view of a pouch for in-vitro diagnostic items according to an eighth embodiment of the present invention.

The pouch for in-vitro diagnostic items according to the eighth embodiment of the present invention has two buffer supporter formation portions 73.

Even in the case of the pouch for in-vitro diagnostic items according to the eighth embodiment of the present invention, on either one of the outer surface of the back sheet of the buffer supporter and the outer surface of the front sheet of the buffer supporter, a left bending position line and a right bending position line may be marked at each buffer supporter formation portion 73.

As described above, according to the embodiments of the present invention, the buffer supporter formation portion is formed by extending in a plate shape from one side of the pocket portion of the diagnosis item 10, a buffer support hole is formed penetratingly in a thickness direction in one area of the buffer supporter formation portion, and the buffer supporter 30 is formed by bending motion in the buffer supporter formation portion separated from the pocket portion of the diagnosis item 10, wherein the buffer supporter 30 includes the buffer-mounted shelf 31 for mounting the buffer 202, the left leg portion 32 for supporting shelf to support the left end of the buffer-mounted shelf 31 against the bottom, and the right leg portion for supporting shelf 33 to support the right end of the buffer-mounted shelf 31 against the bottom. Therefore, there is no need to manufacture the buffer supporter separately from the pouch, to accommodate the buffer supporter means in the pocket portion of the diagnosis item 10 when manufacturing the pouch, and to increase the storage space of the diagnosis item 10a in order to accommodate the buffer supporter means.

In addition, by forming a plurality of buffer supporter formation portions, each buffer 202 may be individually supported at the same time when a plurality of buffers 202 are stored in the pocket portion of the diagnosis item 10.

In addition, the buffer supporter formation portion is configured to include the back sheet of the buffer supporter formed by extending from one side of the back sheet of the diagnosis item pocket and the front sheet of the buffer supporter formed extending from one side of the front sheet of the diagnosis item pocket 12. Therefore, the buffer formation portion may be easily formed by extending from the pocket portion of the diagnosis item 10.

In addition, by marking a left bending position line indicating the left bending motion position and a right bending position line indicating the right bending motion position on either one of the outer surface of the back sheet of the buffer supporter and the outer surface of the front sheet of the buffer supporter, the bending member for forming supporter may be bent at the correct position.

In addition, by configuring the buffer supporter formation portion to further include the bending member for forming supporter provided between the inner surface of the back sheet of the buffer supporter and the inner surface of front sheet of the buffer supporter, or on either one of the outer surface of back sheet of the buffer supporter and the outer surface of the front sheet of the buffer supporter, the buffer 202 may be stably supported.

In addition, by forming concave-shaped bending auxiliary grooves in the bending member for forming supporter to assist the bending motion at the point where the left bending motion and the right bending motion occur, the bending member for forming supporter may be bent at the correct position and may be easily bent.

While the present invention has been particularly shown and described with reference to particular embodiments thereof, it is to be understood that this invention is not limited to the disclosed embodiments, but, on the contrary, various changes may be made by those skilled in the art without departing from scope of the claims.

## Claims

1. A pouch for in-vitro diagnostics items (10) having a pocket portion with a storage space (10a) for the diagnostics items therein, comprising a buffer support formation portion (20; 40; 50; 60; 71; 72; 73), **characterized in that** the buffer support formation portion (20; 40; 50; 60; 71; 72; 73) extends in a plate shape from one side of the pocket portion, one area of the buffer support formation portion (20; 40; 50; 60; 71; 72; 73) comprises a buffer support hole (20a; 40a;50a; 60a) penetrating in a thickness direction, the buffer support formation portion (20; 40; 50; 60; 71; 72; 73) is configured to be separated from the pocket portion and to form a buffer support (30) by bending motion in the buffer support formation portion (20; 40; 50; 60; 71; 72; 73), wherein the buffer support (30) comprises a shelf (31) for mounting the buffer (202), a left leg portion (32) configured to support the left end of the shelf (31) against a floor surface, and a right leg portion (33) configured to support the right end of the shelf (31) against a floor surface.

2. The pouch for in-vitro diagnostic items according to claim 1, wherein the buffer support formation portion (20; 40; 50; 60; 71; 72; 73) is formed in plurality.

3. The pouch for in-vitro diagnostic items according to claim 1, wherein
the pocket portion has a back sheet (11) of the pocket and a front sheet (12) of the pocket coupled to the back sheet (11) of the pocket such that the storage space (10a) of the diagnosis item is formed between the front sheet (12) of the pocket and the back sheet (11) of the pocket;
the buffer support formation portion (20; 40; 50; 60; 71; 72; 73) comprises a back sheet (21;41; 61) of the buffer support (30) formed by extending from one side of the back sheet (11) of the pocket, and a front sheet (22; 42; 62) of the buffer support (30) formed by extending from one side of the front sheet (11) of the pocket and coupled to be superimposed on the back sheet (21;41; 61) of the buffer support (30);
the buffer support hole (20a; 40a;50a; 60a) penetrates the back sheet (21;41; 61) of the buffer support and the front sheet (22; 42; 62) of the buffer support (30); and
the back sheet (21;41; 61) of the buffer support (30) and the front sheet (22; 42; 62) of the buffer support (3) are configured to undergo a left bending motion and a right bending motion to form the buffer support (30).

4. The pouch for in-vitro diagnostic items according to claim 3, wherein
on either one of the outer surface of the back sheet (21;41; 61) of the buffer support and the outer surface of the front sheet (22; 42; 62) of the buffer support (30), a left bending position line (22a; 42a;62a) indicating a left bending motion position and a right bending position line (22b, 42b, 62b) indicating a right bending motion position are respectively marked.

5. The pouch for in-vitro diagnostic items according to claim 3, wherein
the buffer support formation portion (20; 40; 50; 60; 71; 72; 73) further comprises a bending member (23) for forming support provided between the inner surface of the back sheet (21;41; 61) of the buffer support (30) and the inner surface of the front sheet (22; 42; 62) of the buffer support (30), or on either one of the outer surface of the back sheet (21;41; 61) of the buffer support (30) and the outer surface of the front sheet (22;42; 62) of the buffer support (30);
the buffer support hole (20a; 40a;50a; 60a) penetrates the back sheet (21;41; 61) of the buffer support (30), the front sheet (22;42; 62) of the buffer support (30) and the bending member (23) for forming support; and
to form the buffer support (30), the back sheet (21, 41; 61) of the buffer support (30) and the front sheet (22;42; 62) of the buffer support (30) are configured to undergo the left bending motion and the right bending motion, and the bending member (23) for forming support is configured to undergo the left bending motion and right bending motion.

6. The pouch for in-vitro diagnostic items according to claim 3, wherein
the buffer support formation portion (20; 40; 50; 60; 71; 72; 73) further comprises a bending member (23) for forming support provided between the inner surface of the back sheet (21;41; 61) of the buffer support (30) and the inner surface of the front sheet (22;42; 62) of the buffer support (30) or on either one of the outer surface of the back sheet (21;41; 61) of the buffer support (30) and the outer surface of the front sheet (22;42; 62) of the buffer support (30); and
to form the buffer support (30), the back sheet (21;41; 61) of the buffer support and the front sheet (22; 42; 62) of the buffer support (3) are configured to undergo the left bending motion and the right bending motion, and the bending member (23) for forming support is configured to undergo the left bending motion and right bending motion.

7. The pouch for in-vitro diagnostic items according to claim 5 or 6, wherein in the bending member (23) for forming support, concave-shaped bending auxiliary grooves (23a, 43a; 55a) are configured to assist the bending motion at the point where the left bending motion occurs and at the point where the right bending motion occurs.

## Patentansprüche

1. Beutel für In-vitro-Diagnostika (10), der darin einen Taschenabschnitt mit einem Aufbewahrungsbereich (10a) für die Diagnostika aufweist und einen zum Bilden eines Pufferlösungsträgers bestimmten Abschnitt (20; 40; 50; 60; 71; 72; 73) umfasst, **dadurch gekennzeichnet dass**
sich der zum Bilden des Pufferlösungsträgers bestimmte Abschnitt (20; 40; 50; 60; 71; 72; 73) in einer Plattenform von einer Seite des Taschenabschnitts aus erstreckt, wobei ein Bereich des zum Bilden des Pufferlösungsträgers bestimmten Abschnitts (20; 40; 50; 60; 71; 72; 73) ein Pufferlösungsträgerloch (20a; 40a; 50a; 60a) umfasst, das in einer Dickenrichtung hindurchführt, wobei der zum Bilden des Pufferlösungsträgers bestimmte Abschnitt (20; 40; 50; 60; 71; 72; 73) dazu ausgelegt ist, von dem Taschenabschnitt getrennt zu werden und durch eine Biegebewegung in dem zum Bilden des Pufferlösungsträgers bestimmten Abschnitt (20; 40; 50; 60; 71; 72; 73) einen Pufferlösungsträger (30) zu bilden, wobei der Pufferlösungsträger (30) eine Ablage (31) zum Befestigen der Pufferlösung (202) umfasst, wobei ein linker Schenkelabschnitt (32) dazu ausgelegt ist, das linke Ende der Ablage (31) gegen eine Bodenfläche zu tragen, und ein rechter Schenkelabschnitt (33) dazu ausgelegt ist, das rechte Ende der Ablage (31) gegen eine Bodenfläche zu tragen.

2. Beutel für In-vitro-Diagnostika nach Anspruch 1, wobei der zum Bilden des Pufferlösungsträgers bestimmte Abschnitt (20; 40; 50; 60; 71; 72; 73) in einer Vielzahl gebildet ist.

3. Beutel für In-vitro-Diagnostika nach Anspruch 1, wobei
der Taschenabschnitt eine hintere Bahn (11) der Tasche aufweist und eine vordere Bahn (12) der Tasche auf eine solche Weise mit der hinteren Bahn (11) der Tasche gekoppelt ist, dass der Aufbewahrungsbereich (10a) für die Diagnostika zwischen der vorderen Bahn (12) der Tasche und der hinteren Bahn (11) der Tasche gebildet ist,
der zum Bilden des Pufferlösungsträgers bestimmte Abschnitt (20; 40; 50; 60; 71; 72; 73) eine hintere Bahn (21; 41; 61) des Pufferlösungsträgers (30), die dadurch gebildet ist, dass sie sich von einer Seite der hinteren Bahn (11) der Tasche aus erstreckt, und eine vordere Bahn (22; 42; 62) des Pufferlösungsträgers (30) umfasst, die dadurch gebildet ist, dass sie sich von einer Seite der vorderen Bahn (11) der Tasche aus erstreckt und so gekoppelt ist, dass sie über der hinteren Bahn (21; 41; 61) des Pufferlösungsträgers (30) zu liegen kommt,
das Pufferlösungsträgerloch (20a; 40a; 50a; 60a) durch die hintere Bahn (21; 41; 61) des Pufferlösungsträgers und die vordere Bahn (22; 42; 62) des Pufferlösungsträgers (30) hindurchführt und
die hintere Bahn (21; 41; 61) des Pufferlösungsträgers (30) und die vordere Bahn (22; 42; 62) des Pufferlösungsträgers (3) dazu ausgelegt sind, eine Biegebewegung nach links und eine Biegebewegung nach rechts auszuführen, um den Pufferlösungsträger (30) zu bilden.

4. Beutel für In-vitro-Diagnostika nach Anspruch 3, wobei
auf der Außenfläche der hinteren Bahn (21; 41; 61) des Pufferlösungsträgers und der Außenfläche der vorderen Bahn (22; 42; 62) des Pufferlösungsträgers (30) jeweils eine Linksbiegepositionslinie (22a; 42a; 62a), die eine Position für die Biegebewegung nach links angibt, und eine Rechtsbiegepositionslinie (22b; 42b; 62b), die eine Position für die Biegebewegung nach rechts angibt, entsprechend markiert sind.

5. Beutel für In-vitro-Diagnostika nach Anspruch 3, wobei
der zum Bilden des Pufferlösungsträgers bestimmte Abschnitt (20; 40; 50; 60; 71; 72; 73) ferner ein Biegeelement (23) zum Bilden des Trägers umfasst, das zwischen der Innenfläche der hinteren Bahn (21; 41; 61) des Pufferlösungsträgers (30) und der Innenfläche der vorderen Bahn (22; 42; 62) des Pufferlösungsträgers (30) oder jeweils auf der Außenfläche der hinteren Bahn (21; 41; 61) des Pufferlösungsträgers (30) und der Außenfläche der vorderen Bahn (22; 42; 62) des Pufferlösungsträgers (30) bereitgestellt ist,
das Pufferlösungsträgerloch (20a; 40a; 50a; 60a) durch die hintere Bahn (21; 41; 61) des Pufferlösungsträgers (30), die vordere Bahn (22; 42; 62) des Pufferlösungsträgers (30) und das Biegeelement (23) zum Bilden des Trägers hindurchführt, und
um den Pufferlösungsträger (30) zu bilden, die hintere Bahn (21; 41; 61) des Pufferlösungsträgers (30) und die vordere Bahn (22; 42; 62) des Pufferlösungsträgers (30) dazu ausgelegt sind, die Biegebewegung nach links und die Biegebewegung nach rechts auszuführen, und das Biegeelement (23) zum Bilden des Trägers dazu ausgelegt ist, die Biegebewegung nach links und die Biegebewegung nach rechts auszuführen.

6. Beutel für In-vitro-Diagnostika nach Anspruch 3, wobei
der zum Bilden des Pufferlösungsträgers bestimmte Abschnitt (20; 40; 50; 60; 71; 72; 73) ferner ein Biegeelement (23) zum Bilden des Trägers umfasst, das zwischen der Innenfläche der hinteren Bahn (21; 41; 61) des Pufferlösungsträgers (30) und der Innenfläche der vorderen Bahn (22; 42; 62) des Pufferlösungsträgers (30) oder jeweils auf der Außenfläche der hinteren Bahn (21; 41; 61) des Pufferlösungsträgers (30) und der Außenfläche der vorderen Bahn (22; 42; 62) des Pufferlösungsträgers (30) bereitgestellt ist, und
um den Pufferlösungsträger (30) zu bilden, die hintere Bahn (21; 41; 61) des Pufferlösungsträgers und die vordere Bahn (22; 42; 62) des Pufferlösungsträgers (3) dazu ausgelegt sind, die Biegebewegung nach links und die Biegebewegung nach rechts auszuführen, und das Biegeelement (23) zum Bilden des Trägers dazu ausgelegt ist, die Biegebewegung nach links und die Biegebewegung nach rechts auszuführen.

7. Beutel für In-vitro-Diagnostika nach Anspruch 5 oder 6, wobei konkav geformte Biegehilfsrillen (23a; 43a; 55a) in dem Biegeelement (23) zum Bilden des Trägers dazu ausgelegt sind, um an dem Punkt, an dem die Biegebewegung nach links erfolgt, und an dem Punkt, an dem die Biegebewegung nach rechts erfolgt, die Biegebewegung zu unterstützen.

## Revendications

1. Pochette pour articles de diagnostic in vitro (10) ayant une partie poche avec un espace de rangement (10a) pour les articles de diagnostic qui s'y trouvent, comprenant une partie de formation de support de tampon (20 ; 40 ; 50 ; 60 ; 71 ; 72 ; 73), **caractérisée en ce que**
la partie de formation de support de tampon (20 ; 40 ; 50 ; 60 ; 71 ; 72 ; 73) s'étend en forme de plaque à partir d'un côté de la partie poche, une zone de la partie de formation de support de tampon (20 ; 40 ; 50 ; 60 ; 71 ; 72 ; 73) comprend un trou de support de tampon (20a ; 40a ; 50a ; 60a) traversant dans le sens de l'épaisseur, la partie de formation de support de tampon (20 ; 40 ; 50 ; 60 ; 71 ; 72 ; 73) est configurée pour être séparée de la partie poche et pour former un support de tampon (30) par un mouvement de pliage dans la partie de formation de support de tampon (20 ; 40 ; 50 ; 60 ; 71 ; 72 ; 73), dans laquelle le support de tampon (30) comprend une tablette (31) pour tenir le tampon (202), une partie de pied à gauche (32) configurée pour soutenir l'extrémité gauche de la tablette (31) contre une surface de plancher, et une partie de pied à droite (33) configurée pour soutenir l'extrémité droite de la tablette (31) contre une surface de plancher.

2. Pochette pour articles de diagnostic in vitro selon la revendication 1, dans laquelle la partie de formation de support de tampon (20 ; 40 ; 50 ; 60 ; 71 ; 72 ; 73) est formée en plusieurs parties.

3. Pochette pour articles de diagnostic in vitro selon la revendication 1, dans laquelle
la partie poche comporte une feuille arrière (11) de la poche et une feuille avant (12) de la poche couplée à la feuille arrière (11) de la poche de sorte que l'espace de rangement (10a) de l'article de diagnostic est formé entre la feuille avant (12) de la poche et la feuille arrière (11) de la poche ;
la partie de formation de support de tampon (20 ; 40 ; 50 ; 60 ; 71 ; 72 ; 73) comprend une feuille arrière (21 ; 41 ; 61) du support de tampon (30) formée par extension à partir d'un côté de la feuille arrière (11) de la poche, et une feuille avant (22 ; 42 ; 62) du support de tampon (30) formée par extension à partir d'un côté de la feuille avant (11) de la poche et couplée pour être superposée à la feuille arrière (21 ; 41 ; 61) du support de tampon (30) ;
le trou de support de tampon (20a ; 40a ; 50a ; 60a) traverse la feuille arrière (21 ; 41 ; 61) du support de tampon et la feuille avant (22 ; 42 ; 62) du support de tampon (30) ; et
la feuille arrière (21 ; 41 ; 61) du support de tampon (30) et la feuille avant (22 ; 42 ; 62) du support de tampon (3) sont configurées pour pouvoir subir un mouvement de pliage à gauche et un mouvement de pliage à droite afin de former le support de tampon (30).

4. Pochette pour articles de diagnostic in vitro selon la revendication 3, dans laquelle
sur l'une ou l'autre de la surface extérieure de la feuille arrière (21 ; 41 ; 61) du support de tampon et de la surface extérieure de la feuille avant (22 ; 42 ; 62) du support de tampon (30), une ligne de position de pliage à gauche (22a ; 42a ; 62a) indiquant une position de mouvement de pliage à gauche et une ligne de position de pliage à droite (22b, 42b, 62b) indiquant une position de mouvement de pliage à droite sont respectivement marquées.

5. Pochette pour articles de diagnostic in vitro selon la revendication 3, dans laquelle
la partie de formation de support de tampon (20 ; 40 ; 50 ; 60 ; 71 ; 72 ; 73) comprend en outre un élément de pliage (23) servant à former le support, placé entre la surface interne de la feuille arrière (21 ; 41 ; 61) du support de tampon (30) et la surface interne de la feuille avant (22 ; 42 ; 62) du support de tampon (30), ou sur l'une ou l'autre de la surface extérieure de la feuille arrière (21 ; 41 ; 61) du support de tampon (30) et de la surface extérieure de la feuille avant (22 ; 42 ; 62) du support de tampon (30) ;
le trou du support de tampon (20a ; 40a ; 50a ; 60a) traverse la feuille arrière (21 ; 41 ; 61) du support de tampon (30), la feuille avant (22 ; 42 ; 62) du support de tampon (30) et l'élément de pliage (23) servant à former le support ; et
pour former le support de tampon (30), la feuille arrière (21 ; 41 ; 61) du support de tampon (30) et la feuille avant (22 ; 42 ; 62) du support de tampon (30) sont configurées pour pouvoir subir le mouvement de pliage à gauche et le mouvement de pliage à droite, et l'élément de pliage (23) servant à former le support est configuré pour pouvoir subir le mouvement de pliage à gauche et le mouvement de pliage à droite.

6. Pochette pour articles de diagnostic in vitro selon la revendication 3, dans laquelle
la partie de formation de support de tampon (20 ; 40 ; 50 ; 60 ; 71 ; 72 ; 73) comprend en outre un élément de pliage (23) servant à former le support, placé entre la surface interne de la feuille arrière (21 ; 41 ; 61) du support de tampon (30) et la surface interne de la feuille avant (22 ; 42 ; 62) du support de tampon (30), ou sur l'une ou l'autre de la surface extérieure de la feuille arrière (21 ; 41 ; 61) du support de tampon (30) et de la surface extérieure de la feuille avant (22 ; 42 ; 62) du support de tampon (30) ; et
pour former le support de tampon (30), la feuille arrière (21 ; 41 ; 61) du support de tampon et la feuille avant (22 ; 42 ; 62) du support de tampon (3) sont configurées pour pouvoir subir le mouvement de pliage à gauche et le mouvement de pliage à droite, et l'élément de pliage (23) servant à former le support est configuré pour pouvoir subir le mouvement de pliage à gauche et le mouvement de pliage à droite.

7. Pochette pour articles de diagnostic in vitro selon la revendication 5 ou 6, dans laquelle dans l'élément de pliage (23) servant à former le support, des rainures auxiliaires de pliage de forme concave (23a ; 43a ; 55a) sont configurées pour faciliter le pliage à l'endroit où a lieu le mouvement de pliage à gauche et à l'endroit où a lieu le mouvement de pliage à droite.
